**Europäisches Patentamt**

**European Patent Office**

**Office européen des brevets**

(19)

(11) Veröffentlichungsnummer : **0 329 010 B1**

(12) **EUROPÄISCHE PATENTSCHRIFT**

(45) Veröffentlichungstag der Patentschrift :
**06.05.92 Patentblatt 92/19**

(51) Int. Cl.⁵ : **C07C 255/50, A61K 31/275**

(21) Anmeldenummer : **89102202.2**

(22) Anmeldetag : **09.02.89**

(54) **Noremopamil, seine Herstellung und Verwendung.**

(30) Priorität : **19.02.88 DE 3805225**

(43) Veröffentlichungstag der Anmeldung :
**23.08.89 Patentblatt 89/34**

(45) Bekanntmachung des Hinweises auf die
Patenterteilung :
**06.05.92 Patentblatt 92/19**

(84) Benannte Vertragsstaaten :
**AT BE CH DE FR GB IT LI NL**

(56) Entgegenhaltungen :
**EP-A- 0 147 707**
**DE-A- 3 143 356**
**CHEMICAL ABSTRACTS, 11th Collective Index, Bände 96-105, Formulas C20H20-C22H28Zr, 1982-86, Columbus, Ohio, USA; Seite 16069F, Spalte 3, Zeilen 40-42**

(56) Entgegenhaltungen :
**CHEMICAL ABSTRACTS, Band 109, Nr. 3, 18. Juli 1988, Columbus, Ohio, USA; J. WEBER et al: "Effects of phenylalkylamine calciumentry blockers on postischemic energy metabolism in the isolated perfused rat brain: stereoselective action of emopamil", Seite 17, Spalte 2, Zusammenfassung Nr. 16607h**

(73) Patentinhaber : **BASF Aktiengesellschaft**
**Carl-Bosch-Strasse 38**
**W-6700 Ludwigshafen (DE)**

(72) Erfinder : **Hofmann, Hans Peter, Dr.**
**Untere Hart 12**
**W-6703 Limburgerhof (DE)**
Erfinder : **Seitz, Werner, Dr.**
**Bismarckstrasse 22 b**
**W-6831 Plankstadt (DE)**
Erfinder : **Treiber, Hans-Joerg, Dr.**
**Sperberweg 1**
**W-6835 Bruehl (DE)**

## Beschreibung

Die vorliegende Erfindung betrifft racemisches Noremopamil (= 2-Isopropyl-5-phenethylamino-2-phenylvaleronitril) und dessen (S)-Enantiomer sowie deren Verwendung.

Racemisches Noremopamil hat irrtümlicherweise von Chemical Abstracts die REGISTRY-Nummer 86656-29-3 erhalten, obwohl diese Verbindung weder im entsprechenden Chemical Abstracts Zitat (CA 99 (11), 87900 b (1983)) noch in der dazugehörigen Patentanmeldung (DE-OS 31 43 356) aufzufinden ist.

In der EP-OS 147 707 ist das 2-Isopropyl-5-(methylphenethylamino)-2-phenylvaleronitril (im folgenden als Emopamil bezeichnet) und seine Enantiomeren sowie deren Verwendung bei der Bekämpfung von Krankheiten u.a. bei Sauerstoffmangel-Zuständen des Gehirns beschrieben.

Es wurde nun gefunden, daß racemisches Noremopamil und sein (S)-Enantiomer und deren Salze mit physiologisch verträglichen Säuren bei der protektiven Behandlung von hypoxischen Gewebsschädigungen dem Emopamil und dessen Enantiomeren deutlich überlegen sind.

Noremopamil kann nach den in der EP-OS 231003 beschriebenen Verfahren hergestellt werden. Bevorzugt ist die phasentransferkatalysierte Umsetzung von $\alpha$-Isopropylbenzylcyanid mit N-(3-Chlorpropyl)-phenethylamin.

Das (S)-Enantiomer erhält man, indem man das Racemat mit chiralen Säuren umsetzt, das so erhaltene Gemisch der diastereomeren Salze trennt, aus dem reinen diastereomeren Salz die Base freisetzt und diese gegebenenfalls in ihre Salze mit physiologisch verträglichen Säuren überführt.

Beispiele für chirale Säuren sind die optisch aktiven Formen der Campher-10-sulfonsäure, Camphersäure, Weinsäure, Mandelsäure sowie der O,O'-Diacetyl-, O,O'-Dibenzoyl- und O,O'-Di-p-toluoylweinsäure.

Die Trennung der diastereomeren Salze kann in üblicher Weise, z.B. durch fraktionierte Kristallisation oder Säulenchromatographie, erfolgen.

Die Racemattrennung kann auch chromatographisch an chiralen Trägermaterialien erfolgen. Dafür kommen beispielsweise $\beta$-Cyclodextrin, Celluloseester (Acetate, Benzoate, Cinnamate), Cellulosephenylcarbamate, Cellulosetribenzylether, Orosomucoid gebunden an Kieselgel und optisch aktive Polymethylacrylate in Betracht. In diesem Fall erhält man das (S)-Enantiomer direkt, indem man das Racemat über eine Säule aus den genannten Trägermaterialien führt.

(S)-Noremopamil erhält man auch durch Umsetzen von (S)-Emopamil mit Chlorameisensäureester zum Carbamat, das in bekannter Weise (T.W. Greene, "Protective Groups in Organich Synthesis", J. Wiley, New York 1981, S. 223) in das (S)-Noremopamil überführt wird.

Als physiologisch verträgliche Säuren, die sich zur Salzbildung mit Noremopamil und seinen (S)-Enantiomer eignen, seien speziell Schwefelsäure, Phosphorsäure, Weinsäure, Essigsäure, Milchsäure, Maleinsäure, Fumarsäure und insbesondere Salzsäure genannt.

Wie bereits erwähnt, eignet sich Noremopamil und sein (S)-Enantiomer sehr gut zur protektiven Behandlung von Sauerstoffmangel-Zuständen vitaler Organe, insbesondere des Gehirns.

Die deutliche Überlegenheit von Noremopamil und seinem (S)-Enantiomer ließ sich am Modell der normobaren Hypoxie zeigen:

Weibliche Albinomäuse (NMRI, Ivanovas, Kißlegg; Gewicht: 22 bis 28 g) werden - einzeln in Glasröhrchen sitzend - in einer Durchströmungsapparatur einem Gasgemisch von 3,5 % $O_2$ + 96,5 % $N_2$ ausgesetzt, das über ein Rotameter mit einer Durchströmungsgeschwindigkeit von 4 l/min zugeleitet wird. 1 Stunde nach peroraler Vorbehandlung wird die Überlebenszeit der Tiere ermittelt, entsprechend dem Abstand zwischen Beginn der Durchströmung und dem Atemstillstand. Unter 6 parallel geprüften Tieren befindet sich stets 1 Kontrolltier.

Mittels linearer Regression wird aus den - auf Placebo-behandelte Kontrolltiere bezogenen - Überlebenszeiten substanzbehandelter Tiere und den logarithmierten Dosen (mg/kg) als $ED_{33\%}$ die Dosis in mg/kg (mit 95 % Konfidenzintervall) ermittelt, welche eine 33 %ige Verlängerung der Überlebenszeit der behandelten Tiere im Vergleich zum Kontrollkollektiv bewirkt.

Tabelle 1 belegt, daß Noremopamil und dessen (S)-Enantiomer dem Emopamil und dessen (S)-Enantiomer in der cerebroprotektiven Wirkung deutlich überlegen sind.

Tabelle 1

Vergleich der cerebroprotektiven Wirkung

Verlängerung der Überlebenszeit unter normobarer hypoxischer Hypoxie (3,5 % $O_2$ + 96,5 % $N_2$) 1 Stunde nach peroraler Vorbehandlung an der Maus; $ED_{33\%}$ ist die Dosis, welche eine 33 %ige Verlängerung der Überlebenszeit im Vergleich zu Placebo-behandelten Kontrolltieren bewirkt.

| Substanz | $ED_{33\%}$ mg/kg p.o. |
|----------|------------------------|
| Noremopamil | 10,3 |
| (S)-Noremopamil | 4,2 |
| Emopamil | 51,1 |
| (S)-Emopamil | 32,1 |

Insbesondere eignen sich Noremopamil und sein (S)-Enantiomeres zur Behandlung von akuten und chronischen Sauerstoffmangel-Zuständen vitaler Organe, insbesondere des Gehirns. Hierunter sind akute hypoxische bzw. ischämische Zustände zu verstehen, die z.B.infolge Hirninfarkt, Schädel-Hirn-Trauma oder Gefäßspasmen sowie infolge von Herz-Kreislauf-Versagen, z.B. bei Herzstillstand, kardialen Arrhythmien oder Kreislaufschock auftreten. Als Krankheitsbilder mit chronischen Sauerstoffmangel-Zuständen kommen z.B.in Betracht: transitorische ischämische Attacken (TIAs) und prolongierte reversible ischämische neurologische Defizite (PRINDs), aber auch Multiinfarktdemenz und atherosklerotische Demenz, außerdem Migräne vaskulären Ursprungs.

Noremopamil und sein (S)-Enantiomer können in üblicher Weise oral oder parenteral (intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 50 mg/kg Körpergewicht bei oraler Gabe und zwischen etwa 0,1 und 5 mg/kg Körpergewicht bei parenteraler Gabe.

Noremopamil und sein (S)-Enantiomer können in den gebräuchlichen galenischen Applikationsformen fest oder flüssig angewendet werden, z.B. als Tabletten, Filmtabletten, Kapseln, Pulver, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gewichtsprozent.

Die folgenden Beispiele erläutern die Erfindung.

Beispiel 1

2-Isopropyl-5-phenethylamino-2-phenylvaleronitril

In einem Dreihalskolben, der mit Rührwerk, Tropftrichter und Rückflußkühler ausgerüstet war, wurden 15,9 g (0,1 Mol) $\alpha$-Isopropyl-benzylcyanid in 20 ml Toluol gelöst. Zu dieser Lösung gab man 32,5 g 85 %iges technisches Kaliumhydroxidpulver und 0,2 g Tris-(3,6-dioxaheptyl)amin. Unter kräftigem Rühren wurde das Reaktionsgemisch auf 80°C erwärmt und bei dieser Temperatur beginnend eine Lösung von 19,8 g (0,1 Mol) N-(3-Chlorpropyl)-phenethylamin in 20 ml Toluol in dem Maße zugetropft, daß eine Reaktionstemperatur von 85°C nicht überschritten wurde. Nach beendeter Zugabe wurde 3 h bei 85 bis 90°C nachgerührt.

Die erkaltete Reaktionsmischung wurde mit 100 ml Wasser und 100 ml Toluol versetzt. Die Toluolphase wurde abgetrennt und mehrmals mit Wasser gewaschen. Nach Trocknen und Abziehen des Lösungsmittels verblieben 30 g öliger Rückstand, der in 200 ml Essigester gelöst und anschließend mit ethanolischer Salzsäure versetzt wurde. Nach Stehen über Nacht wurden 32 g (92 %) Hydrochlorid, Fp. 163 bis 164°C, isoliert.

Beispiel 2

(S)-2-Isopropyl-5-phenethylamino-2-phenylvaleronitril

38,4 g (0,12 Mol) racemisches 2-Isopropyl-5-phenethylamino-2-phenylvaleronitril und 48,5 g (0,12 Mol) (+)-O,O'-Di-4-toluoyl-D-weinsäure wurden unter Erwärmen in 400 ml Isopropanol gelöst. Das über Nacht ausgefallene Kristallisat wurde abgesaugt und zweimal aus einem Ethanol-Wasser-Gemisch (3:1) umkristallisiert. Der gefundene Drehwert von $[\alpha]_{589}^{20}$ = +46,0 (Methanol, c = 10 mg/ml) veränderte sich durch nochmalige Kristallisation nicht. Die aus dem Salz freigesetzte Base wurde in 100 ml Essigester gelöst und mit ethanolischer Salzsäure versetzt bis der pH-Wert 3 betrug. Nach Absaugen und Trocknen erhielt man 12,8 g des Hydrochlorids, Fp. 178 bis 180°C, $[\alpha]_{589}^{20}$ = -10,2 (Ethanol, c = 10 mg/ml).

Beispiel 3

(S)-2-Isopropyl-5-phenethylamino-2-phenylvaleronitril

Zu einer Lösung von 6 ml (66 mMol) Chlorameisensäurevinylester in 30 ml Dichlormethan tropfte man bei 0°C eine Lösung von 17 g (50 mMol) (S)-2-Isopropyl-5-(methylphenethylamino)-2-phenylvaleronitril in 50 ml Dichlormethan. Danach ließ man die Temperatur auf Raumtemperatur ansteigen und erwärmte 1 h auf 80°C.

Nach Abdestillieren des Lösungsmittels wurde der Rückstand in 100 ml n-Hexan aufgenommen und mit 0,5 %iger wäßriger Amidosulfonsäure extrahiert. Nach Waschen mit verdünnter Kaliumcarbonatlösung und Trocknen über Natriumsulfat wurde das Lösungsmittel abdestilliert und der Rückstand in 100 ml trockenem Dichlormethan aufgenommen. Die Lösung wurde bei 0°C mit Chlorwasserstoff gesättigt und anschließend 4 h bei Raumtemperatur stehen gelassen. Nach Abdestillieren des Lösungsmittels wurde der Rückstand in 150 ml Methanol aufgenommen und 30 min auf 50°C erwärmt. Das Methanol wurde abdestilliert und der Rückstand mit 30 ml Wasser versetzt. Es kristallisierten 8 g Hydrochlorid, Fp. 178 bis 180°C, $[\alpha]_{589}^{20}$ = -10,2 (Ethanol, c = 10 mg/ml), aus.

Beispiel 4

Auf einer Tablettenpresse werden in üblicher Weise Tabletten folgender Zusammensetzung gepreßt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 Ziger Kleister) |

Beispiel 5

In üblicher Weise werden Dragees folgender Zusammensetzung hergestellt:

| | |
|---|---|
| 20 mg | Substanz des Beispiels 2 |
| 60 mg | Kernmasse |
| 60 mg | Verzuckerungsmasse |

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 6

10 g Substanz des Beispiels 3 werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche**

1. 2-Isopropyl-5-phenethylamino-2-phenylvaleronitril und dessen Salze mit physiologisch verträglichen Säuren.

2. (S)-2-Isopropyl-5-phenethylamino-2-phenylvaleronitril und dessen Salze mit physiologisch verträglichen Säuren.

3. 2-Isopropyl-5-phenethylamino-2-phenylvaleronitril und dessen Salze mit physiologisch verträglichen Säuren zur Verwendung als Arzneimittel.

4. (S)-2-Isopropyl-5-phenethylamino-2-phenylvaleronitril und dessen Salze mit physiologisch verträglichen Säuren zur Verwendung als Arzneimittel.

5. Verwendung von 2-Isopropyl-5-phenethylamino-2-phenylvaleronitril und dessen Salzen mit physiologisch verträglichen Säuren zur Herstellung von Arzneimitteln zur Bekämpfung von Sauerstoffmangel-Zuständen.

6. Verwendung von (S)-2-Isopropyl-5-phenethylamino-2-phenylvaleronitril und dessen Salzen mit physiologisch verträglichen Säuren zur Herstellung von Arzneimitteln zur Bekämpfung von Sauerstoffmangel-Zuständen.

## Claims

1. 2-Isopropyl-5-phenethylamino-2-phenylvaleronitrile and salts thereof with physiologically tolerated acids.

2. (S)-2-Isopropyl-5-phenethylamino-2-phenylvaleronitrile and salts thereof with physiologically tolerated acids.

3. 2-Isopropyl-5-phenethylamino-2-phenylvaleronitrile and salts thereof with physiologically tolerated acids for use as drugs.

4. (S)-2-Isopropyl-5-phenethylamino-2-phenylvaleronitrile and salts thereof with physiologically tolerated acids for use as drugs.

5. Use of 2-isopropyl-5-phenethylamino-2 -phenylvaleronitrile and salts thereof with physiologically tolerated acids for the preparation of drugs for the treatment of oxygen deficiency conditions.

6. Use of (S)-2-isopropyl-5-phenethylamino-2-phenylvaleronitrile and salts thereof with physiologically tolerated acids for the preparation of drugs for the treatment of oxygen deficiency conditions.

## Revendications

1. 2-Isopropyl-5-phénétylamino-2-phénylvaléronitrile et ses sels avec des acides physiologiquement acceptables.

2. 2-Isopropyl-5-phénétylamino-2-phénylvaléronitrile-S et ses sels avec des acides physiologiquement acceptables.

3. 2-Isopropyl-5-phénétylamino-2-phénylvaléronitrile et ses sels avec des acides physiologiquement acceptables pour l'utilisation commes médicaments.

4. 2-Isopropyl-5-phénétylamino-2-phénylvaléronitrile-S et ses sels avec des acides physiologiquement acceptables pour l'utilisation comme médicaments.

5. Utilisation de 2-isopropyl-5-phénétylamino-2-phénylvaléronitrile et de ses sels avec des acides physiologiquement acceptables pour la préparation de médicaments pour la lutte contre les états de manque d'oxygène.

6. Utilisation de 2-isopropyl-5-phénétylamino-2-phénylvaléronitrile-S et des ses sels avec des acides physiologiquement acceptables pour la préparation de médicaments pour la lutte contre les états de manque d'oxygène.